Europäisches Patentamt

European Patent Office

Office européen des brevets

(11) Veröffentlichungsnummer: **0 108 246**
**B1**

(12) **EUROPÄISCHE PATENTSCHRIFT**

(45) Veröffentlichungstag der Patentschrift:
29.04.87

(21) Anmeldenummer: **83109840.5**

(22) Anmeldetag: **01.10.83**

(51) Int. Cl.⁴: **C 08 F 220/56,** C 08 F 2/44,
**C 12 N 11/04**

(54) **Verfahren zur Herstellung von perlförmigen Biokatalysatoren und ihre Verwendung.**

(30) Priorität: **08.10.82 DE 3237341**

(43) Veröffentlichungstag der Anmeldung:
**16.05.84 Patentblatt 84/20**

(45) Bekanntmachung des Hinweises auf die Patenterteilung:
**29.04.87 Patentblatt 87/18**

(84) Benannte Vertragsstaaten:
**AT BE CH DE FR GB IT LI LU NL SE**

(56) Entgegenhaltungen:
**DE-A-2 343 633**
**DE-A-2 805 607**

(73) Patentinhaber: **HOECHST AKTIENGESELLSCHAFT,
Postfach 80 03 20, D-6230 Frankfurt am Main 80
(DE)**

(72) Erfinder: **Keller, Reinhold, Dr., Wiesenweg 5,
D-6232 Bad Soden am Taunus (DE)**
Erfinder: **Siegemund, Günter, Dr., Frankfurter
Strasse 21, D-6238 Hofheim am Taunus (DE)**

## Beschreibung

Biokatalysatoren sind für die direkte Gewinnung von primären und sekundären Stoffwechselprodukten von zunehmender Bedeutung. Beispiele der Praxis, für welche technologisches Interesse besteht, sind:

Gewinnung von Fructose aus Glucose mittels Glucoseisomerase, Herstellung von 6-Aminopenicillansäure aus Penicillin G mittels Penicillin-Acylase, Herstellung von L-Asparaginsäure aus Ammoniumfumarat mittels E.coli sowie Herstellung von Apfelsäure aus Fumarsäure mit Brevibakterium ammoniagenes-Zellen.

Unter dem Begriff "Biokatalysator" wird in der technischen Mikrobiologie ein mittels eines makroskopischen Trägers fixiertes biologisches System aus ganzzelligen Mikroorganismen oder Enzymen verstanden.

In jüngerer Zeit wird dem Einsatz von fixierten Mikroorganismen als Biokatalysator aus Gründen der Herstellungskosten und unter dem Gesichtspunkt von Mehrenzymreaktionen der Vorzug gegeben. Die Herstellung der Biokatalysatoren erfolgt im wesentlichen durch physikalischen Einschluß des Mikroorganismus in eine Polymermatrix. Zur Herstellung derartiger Matrices wurden nach unterschiedlichen Verfahrensschemata die verschiedensten Materialien verwendet, wobei sich Polymerisate niedermolekularer ungesättigter Carbonsäuren wie Acrylsäure sowie Derivate derselben besonders bewährt haben.

Perlförmige Mischpolymerisate als Träger für biologisch aktive Substanzen sind aus der DE-OS 2 343 633 bekannt. Nach dem dort beschriebenen Verfahren wird ein radikalisch polymerisierbares Monomeres, das eine mit primären Aminogruppen oder Hydroxygruppen von biologisch aktiven Substanzen unter Ausbildung einer kovalenten Bindung reagierende Gruppe aufweist, beispielsweise Acrylamid, mit einer Verbindung mit wenigstens 2 radikalisch polymerisierbaren Kohlenstoff-Doppelbindungen wie Methylen-bis-acrylamid oder -methacrylamid in einem hydrophoben organischen Lösemittel radikalisch polymerisiert. Die wäßrige Lösung der biologisch aktiven Komponente, vorzugsweise ein Enzym, wird in einem weiteren Schritt zugegeben.

Nach der DE-OS 2 805 607 wird nicht nur diese getrennte Zugabe der biologisch aktiven Komponente, in diesem Falle Mikroorganismen, vermieden, sondern es sollen auch mechanisch stabilere Produkte erhalten werden. Hierzu erfolgt die Perlpolymerisation in Anwesenheit der Zellen in wäßrigorganischer Suspension, wobei Methacrylamid und Methylen-bisacrylamid radikalisch polymerisiert werden. Kurz bevor der Gelpunkt des Systems erreicht wird, werden die Mikroorganismen zugegeben und eine gleichmäßige Verteilung herbeigeführt. Die Bestimmung des Gelpunkts erfolgt hierbei zweckmäßig dadurch, daß in einem zeitlich etwas vorgezogenen Parallelansatz die Zeit vom Beginn der Polymerisation bis zum Eintritt der Gelbildung unter den Parametern der Herstellung dieses Polymerisationsansatzes ermittelt wird. Als Suspensions-Flüssigkeit dient vorzugsweise ein Diester der Terephthalsäure mit Alkanolen mit 1 bis 10 Kohlenstoffatomen.

Diese genannten Verfahren sind ziemlich aufwendig. Es wurde nun gefunden, daß in einem einfachen Verfahren eine Vielzahl von Mikroorganismen in einen perlförmigen Biokatalysator durch radikalische Polymerisation des Amids einer niedermolekularen ungesättigten Carbonsäure und Methylenbisacrylamid in wäßrig-organischer Suspension eingebaut werden kann, wenn das Amid Acrylamid ist und wenn die organische Phase im wesentlichen aus einer hochfluorierten Kohlenstoffverbindung besteht.

"Im wesentlichen" soll hierbei besagen, daß neben der hochfluorierten Kohlenstoffverbindung auch andere organische Verbindungen zugegen sein können, die die Reaktion nicht stören und natürlich auch den Biokatalysator nicht beeinträchtigen. So sind im Falle lebender Organismen Zellgifte selbstverständlich ausgeschlossen. Eingeschlossen sind hingegen Hilfsstoffe wie Polymerisations-Initiatoren oder oberflächenaktive Substanzen. Im folgenden wird deshalb der Einfachheit halber der Begriff "hochfluorierte Kohlenstoffverbindung(en)" verwendet, ohne daß hiermit eine Einschränkung verbunden ist.

Nach den Angaben der DE-OS 2 805 607 war nicht zu erwarten, daß man mit Acrylamid zu brauchbaren perlförmigen Biokatalysatoren kommt. Weiterhin ist überraschend, daß unter Verwendung der hochfluorierten Kohlenstoffverbindungen in der organischen Phase das Polymerisationsverfahren wesentlich vereinfacht werden konnte. Nach den Angaben der DE-OS 2 343 633 war andererseits nicht zu erwarten, daß bei einer radikalischen Polymerisation von Acrylamid und Methylen-bis-acrylamid ein perlförmiger Biokatalysator hergestellt werden kann, der Zellen enthält, wenn die Polymerisation in einem wasserhaltigen Medium erfolgt, dessen organische Phase eine hochfluorierte Kohlenstoffverbindung enthält.

In folgenden werden bevorzugte Ausgestaltungen der Erfindung näher erläutert:

Die Mikroorganismen werden als Feucht-Zellmasse vermehrungsfähiger oder toter Organismen eingesetzt, zweckmäßig als abzentrifugierte und gewaschene Zellmasse, die in physiologischer Kochsalzlösung aufgenommen wird. Hierzu werden die Monomeren Acrylamid und Methylen-bis-acrylamid gegeben, unter kräftigem Rühren in der hochfluorierten Kohlenstoffverbindung suspendiert und die Polymerisation gestartet.

Als Katalysator dienen bevorzugt Persulfate wie Kaliumpersulfat oder Ammoniumpersulfat in Kombination mit einem Initiator wie β-Dimethylamio-propionitril oder N,N,N',N'-Tetramethylethylendiamin.

Die Polymerisationstemperatur liegt zweckmäßig nicht oberhalb der Raumtemperatur, vorzugsweise bei 5 - 20°C, insbesondere bei 10 - 15°C.

Das Verhältnis von wäßriger zu organischer Phase kann in relativ weiten Grenzen schwanken. Zweckmäßig ist ein Volumenverhältnis von 1 : 3 bis 1 : 15, bevorzugt 1 : 6 bis 1 : 10.

Als organische Phase können alle im genannten Temperaturbereich flüssigen hochfluorierten Fluorkohlenstoffverbindungen eingesetzt werden. Maßgeblich für die Eignung ist lediglich, daß mindestens die Hälfte aller Bindungen im Molekül Kohlenstoff-Fluor-Bindungen darstellen. Selbstverständlich kommen auch Mischungen geeigneter Verbindungen in Betracht, beispielsweise unterschiedlich hoch fluorierter Verbindungen desselben Strukturtyps, aber auch Mischungen von Verbindungen unterschiedlicher Strukturklassen.

Der Siedepunkt der hochfluorierten Kohlenstoffverbindungen ist demgegenüber unkritisch. Bei der Verfahrenstemperatur an sich gasförmige Verbindungen erfordern die Anwendung von Druck und sind deshalb weniger zweckmäßig. Verbindungen mit hohen Siedepunkten sind im allgemeinen relativ viskos, weshalb Produkte mit Siedepunkten von 40-300°C, insbesondere 90 - 200°C, jeweils bei Normaldruck, bevorzugt sind.

Bevorzugt sind perfluorierte gesättigte und ungesättigte, lineare oder cyclische Kohlenwasserstoffe wie Perfluornonan, 1-Perfluornonen, 1,2-Bis-(perfluor-n-butyl)-ethylen 1,2-Bis-(perfluor-n-hexyl)-ethylen, 1,2-Bis-(perfluor-n-octyl)-ethylen, Perfluordecahydronaphthalin, Perfluor-1-methyldecahydronaphthalin, Perfluor-1,3-dimethylcyclohexan, Perfluor-methyladamantan oder Perfluor-dimethyladamantan sowie andere Halogenatome enthaltende hochfluorierte Kohlenwasserstoffe wie 2,3-Dichlor-perfluor-2-methylpentan, 1-Brom-perfluoroctan oder 1-Chlor-perfluoroctan, Ether wie Perfluor-2-(n-propoxy)-propyl-1,2,2,2-tetrafluorethylether, niedere Perfluor-polyalkoxypropyl-1,2,2,2-tetrafluorethylether, Perfluorbutyltetrahydrofuran, Perfluorpropyltetrahydrofuran, Perfluor-1,4-bis-(isopropoxy)-butan oder Perfluor-1,8-bis-(isopropoxy)-octan, weiterhin Amine wie Perfluor-tri-n-butylamin·oder Perfluor-tri-n-propylamin.

Zur Stabilisierung der Suspension kann der Einsatz eines Emulgators vorteilhaft sein, wobei zweckmäßig pro Liter hochfluorierter Kohlenstoffverbindung etwa 2 ml eines nichtionischen Emulgators zugegeben werden.

Die Polymerisation erfordert im allgemeinen nur wenige Minuten. Die erhaltenen Biokatalysator-Kügelchen weisen im allgemeinen einen Durchmesser von ungefähr 2 - 3 mm auf und sind deshalb leicht abtrennbar. Zweckmäßig erfolgt ihre Isolierung durch Filtration und Waschen mit Wasser oder bevorzugt mit physiologischer Kochsalzlösung.

Die Perlform des Biokatalysators gestattet eine sehr gute Raum-Zeit-Ausbeute und bedingt eine hohe Stabilität und Aktivität. Es ist somit ein universeller Einsatz in den verschiedensten Reaktortypen möglich. Die Erfindung betrifft deshalb auch die Verwendung der erfindungsgemäß hergestellten Katalysatoren in biotechnischen Verfahren.

In den folgenden Beispielen beziehen sich Prozentangaben auf das Gewicht, sofern nichts anderes angegeben ist.

## Beispiel 1

30 g abzentrifugierte feuchte Lactobacillus bulgaricus-Zellen wurden mit 85,5 g Acrylamid und 4,5 g N',-Methylen-bis-acrylamid in 300 ml physiologischer Kochsalzlösung aufgenommen und unter kräftigem Rühren zu einer Mischung von 1 l Perfluornonan und 2 ml eines nichtionischen Tensids, die zuvor mit Stickstoff von Sauerstoff befreit wurde, gegeben. Bei 10°C wurde die Polymerisation mit 0,3 g Ammoniumpersulfat, gelöst in 2 ml 0,9 %iger Kochsalzlösung, und 0,5 ml N,N,N',N'-Tetramethylethylendiamin gestartet. Nach etwa 5 Minuten war die Reaktion beendet. Die erhaltenen Perlen wurden abfiltriert und mit Physiologischer Kochsalzlösung gut gewaschen.

Zur Bestimmung der biologischen Aktivität des Biokatalysators wurde ein Teil der Perlen in Glucose-Lösung gegeben und titrimetrisch die Menge an gebildeter Milchsäure ermittelt. Der Vergleich mit der gleichen Menge freier Zellen zeigte, daß der Biokatalysator 65 % der ursprünglichen Aktivität besaß.

Nach 70 Tagen Lagerung in 0,9 %iger Kochsalzlösung bei 5°C besaß dieser Katalysator noch etwa 50 % der ursprünglichen Aktivität. Unter gleichen Bedingungen gelagerte freie Zellen waren bereits nach 14 Tagen unbrauchbar.

## Beispiel 2

60 g abzentrifugierte feuchte Hefe-Zellen wurden zusammen mit 171 g Acrylamid und 9,0 g N,N'-Methylen-bis-acrylamid in 500 ml physiologischer Kochsalzlösung aufgenommen und unter kräftigem Rühren zu einer Mischung von 2 l Perfluornonen -(1) und 2 ml eines nichtionischen Tensids, die durch Durchleiten von Stickstoff von Sauerstoff befreit worden war, gegeben. Die Polymerisation wurde bei 10°C mit 0,3 g Ammoniumpersulfat, gelöst in 2 ml 0,9 %iger Kochsalzlösung, und 0,5 ml N,N,N',N'-Tetramethylethylendiamin gestartet. Nach etwa 5 Minuten war die Reaktion beendet. Die erhaltenen Biokatalysator-Perlen wurden abfiltriert und mit physiologischer Kochsalzlösung gut gewaschen.

Zur Bestimmung der biologischen aktivität wurde ein Teil der Perlen in ein geeignetes Nährmedium gegeben und die Bildung von Ethanol gaschromatographisch bestimmt. Der Vergleich mit freien Hefezellen zeigte, daß durch das Immobilisieren keine Aktivität verloren ging.

## Beispiel 3

50 g abzentrifugierte E. coli-Zellen (ATCC 11 105) wurden zusammen mit 50 g Acrylamid und 2,5 g N,N'-Methylen-bis-acrylamid in 300 ml physiologischer Kochsalzlösung aufgenommen und unter kräftigem Rühren zu einer Mischung von 1,5 l 2,3-Dichlorperfluor-2-methylpentan und 1 ml eines nichtionischen Tensids, die durch Durchleiten von Stickstoff von Sauerstoff befreit worden war, gegeben. Die Polymerisation wurde bei 10° C mit 0,3 g Ammoniumpersulfat, gelöst in 2 ml 0,9 %iger Kochsalzlösung, und 0,5 ml N,N,N',N'-Tetramethylethylendiamin gestartet. Nach 5 Minuten war die Reaktion beendet. Die erhaltenen Biokatalysator-Perlen wurden abfiltriert und mit physiologischer Kochsalzlösung gut gewaschen.

Zur Bestimmung der biologischen Aktivität wurde ein Teil der Perlen in Penicillin-G-Lösung gegeben und bei pH 7,6 titrimetrisch die freiwerdende 6-Aminopenicillansäure ermittelt. Der Vergleich mit der gleichen Menge freier Zellen zeigte, daß der Biokatalysator 41 % der ursprünglichen Aktivität besaß.

## Beispiel 4

30 g abzentrifugierte Hefe-Feuchtmasse (Saccharomyces cerevisiae) wurden zusammen mit 8,0 g Acrylamid und 10 g N,N'-Methylen-bis-acrylamid in 250 ml physiologischer Kochsalzlösung aufgenommen und unter kräftigem Rühren zu einer Mischung von 1 l Perfluornonan und 1 ml eines nichtionisehen Tensids, die durch Durchleiten von Stickstoff von Sauerstoff befreit worden war, gegeben. Die Polymerisation wurde bei 10° C mit 0,3 g Ammoniumpersulfat, gelöst in 2 ml 0,9%iger Kochsalzlösung, und 0,5 ml N,N,N',N'-Tetramethylethylendiamin gestartet. Nach etwa 5 Minuten war die Reaktion beendet. Die erhaltenen Biokatalysator-Perlen wurden abfiltriert und mit physiologischer Kochsalzlösung gut gewaschen.

Zur Bestimmung der biologischen Aktivität wurde ein Teil der Perlen in ein geeignetes Nährmedium, welches 5'-Adenosinmonophosphat enthielt, gegeben und die Bildung von ATP bestimmt.

## Patentansprüche

1. Verfahren zur Herstellung eines, einen Mikroorganismus enthaltenden, perlförmigen Biokatalysators durch radikalische Polymerisation zwischen einem Amid einer niedermolekularen ungesättigten Carbonsäure und Methylen-bis-acrylamid in wäßrig-organischer Suspension, dadurch gekennzeichnet, daß

a) das Amid Acrylamid ist,

b) die organische Phase der genannten Suspension im wesentlichen aus einer hochfluorierten Kohlenstoffverbindung besteht und

c) der Mikroorganismus den genannten Monomeren in genannter Suspension zugemischt wird, bevor die Polymerisation gestartet wird.

2. Verfahren nach Anspruch 1, dadurch gekennzeichnet, daß der Mikroorganismus als Feucht-Zellmasse zugemischt wird.

3. Verfahren nach Anspruch 1 oder 2, dadurch gekennzeichnet, daß die Polymerisation mit einem Persulfat gestartet wird.

4. Verfahren nach einem oder mehreren der Ansprüche 1 - 3, dadurch gekennzeichnet, daß die Polymerisationstemperatur nicht oberhalb der Raumtemperatur, vorzugsweise bei 5-20° C, insbesondere bei 10-15° C, liegt.

5. Verfahren nach einem oder mehreren der Ansprüche 1 - 4, dadurch gekennzeichnet, daß das Volumenverhältnis der wäßrigen zur organischen Phase 1 : 3 bis 1 : 15, vorzugsweise 1: 6 bis 1 : 10, beträgt.

6. Verfahren nach einem oder mehreren der Ansprüche 1 - 5, dadurch gekennzeichnet, daß die hochfluorierte Verbindung bei Normaldruck einen Siedepunkt von 40 - 300° C, vorzugsweise 90 - 200° C, aufweist.

7. Verfahren nach einem oder mehreren der Ansprüche 1 - 6, dadurch gekennzeichnet, daß ein Polymerisations-Initiator zugesetzt wird.

8. Verfahren nach einem oder mehreren der Ansprüche 1 - 7, dadurch gekennzeichnet, daß eine oberflächenaktive Verbindung zugesetzt wird.

9. Verfahren nach einem oder mehreren der Ansprüche 1 - 8, dadurch gekennzeichnet, daß die hochfluorierte Kohlenstoffverbindung ein perfluorierter Kohlenwasserstoff ist.

10. Verwendung der nach einem oder mehreren der Ansprüche 1 - 9 erhaltenen Katalysatoren in biotechnischen Verfahren.

## Claims

1. Process for the production of a bead form biocatalyst enclosing a microorganism by radical polymerisation reaction between an amide of a low molecular weight unsaturated carbocyclic acid and methylene-bis-acrylamide in aqueous-organic suspension wherein

a) the amide is acrylamide,

b) the organic phase of said suspension consists essentially of a highly fluorinated carbon compound and

c) the microorganism is mixed with said suspension before polymerisation is started.

2. The process as claimed in claim 1, wherein the microorganism is mixed with said suspension as wet cell agglomerate.

3. The process as claimed in claim 1 or 2, wherein the polymerization is started with a persulfate.

4. The process as claimed in one or more of claims 1 to 3, wherein the polymerization temperature is not above room temperature, preferably 5 to 20°C, especially 10 to 15°C.

5. The process as claimed in one or more of claims 1 to 4, wherein the ratio of the volume of the aqueous to the organic phase is 1:3 to 1:15, preferably 1:6 to 1:10.

6. The process as claimed in one or more of claims 1 to 5, wherein the highly fluorinated compound is a liquid having a boiling point under normal pressure of 40 to 300°C, preferably 90 to 100°C.

7. The process as claimed in one or more of claims 1 to 6, wherein a polymerization initiator is added.

8. The process as claimed in one or more of claims 1 to 7, wherein a surface-active compound is added.

9. The process as claimed in one or more of claims 1 to 8, wherein the highly fluorinated carbon compound is a liquid perfluorinated hydrocarbon.

10. Use of the biocatalyst as obtained in one or more of the preceding processes in biotechnological procedures.

**Revendications**

1. Procédé de fabrication d'un biocatalyseur en forme de perles contenant un microorganisme par polymérisation radicalaire d'un amide d'un acide carboxylique insaturé à faible poids moléculaire et de méthylène-bis-acrylamide en suspension aquo-organique, caractérisé en ce que :

a) l'amide est l'acrylamide,

b) la phase organique de la suspension indiquée est constituée essentiellement d'un composé carboné à haut degré de fluoration et

c) le microorganisme est mélangé aux monomères indiqués dans la suspension indiquée avant que l'on ne commence la polymérisation.

2. Procédé selon la revendication 1, caractérisé en ce que le microorganisme est ajouté sous forme d'une masse de cellules humides.

3. Procédé selon la revendication 1 ou 2, caractérisé en ce que la polymérisation est amorcée avec un persulfate.

4. Procédé selon une ou plusieurs des revendications 1 à 3, caractérisé en ce que la température de polymérisation n'est pas supérieure à la température ambiante, et de préférence est de 5 à 20°C, en particulier de 10 à 15°C.

5. Procédé selon une ou plusieurs des revendications 1 à 4, caractérisé en ce que le rapport en volume de la phase aqueuse à la phase organique est de 1:3 à 1:15, de préférence de 1:6 à 1:10.

6. Procédé selon une ou plusieurs des revendications 1 à 5, caractérisé en ce que le composé à haut degré de fluoration présente sous la pression normale un point d'ébullition de 40 à 300°C, de préférence de 90 à 200°C.

7. Procédé selon une ou plusieurs des revendications 1 à 6, caractérisé en ce que l'on ajoute un initiateur de polymérisation.

8. Procédé selon l'une ou plusieurs des revendications 1 à 7, caractérisé en ce que l'on ajoute un composé tensio-actif.

9. Procédé selon l'une ou plusieurs des revendications 1 à 8, caractérisé en ce que le composé carboné à haut degré de fluoration est un hydrocarbure perfluoré.

10. Utilisation des catalyseurs selon l'une ou plusieurs des revendications 1 à 9 dans des procédés biotechnologiques.